**Europäisches Patentamt**

**European Patent Office**

**Office européen des brevets**

(19)

(11) Publication number : **0 382 210 B1**

## (12) EUROPEAN PATENT SPECIFICATION

(45) Date of publication of patent specification :
20.10.93 Bulletin 93/42

(51) Int. Cl.⁵ : **A61K 31/73**

(21) Application number : **90102485.1**

(22) Date of filing : **08.02.90**

(54) **The use of deacetylated chitin for preparing a medicament for the treatment of inflammatory skin diseases.**

(30) Priority : **08.02.89 JP 29489/89**

(43) Date of publication of application :
**16.08.90 Bulletin 90/33**

(45) Publication of the grant of the patent :
**20.10.93 Bulletin 93/42**

(84) Designated Contracting States :
**DE FR GB**

(56) References cited :
**EP-A- 0 171 254**
**FR-A- 2 609 397**
**GB-A- 2 129 300**
**GB-A- 2 144 630**
**US-A- 3 903 268**
**US-A- 4 363 801**
**J.P. ZIKAKIS: "Chitin, chitosan, and related enzymes", 1984, pages 119-133, Academic Press, Inc., Orlando, FL, US; G.G. ALLEN et al.: "Biomedical applications of chitin and chitosan"**

(73) Proprietor : **UNITIKA LTD.**
**No. 50, Higashihonmachi 1-chome**
**Amagasaki-shi Hyogo (JP)**

(72) Inventor : **Horiuchi, Yasuhiro**
**9108-2, Doai, Hazaki-cho**
**Kashima-gun, Ibaraki (JP)**
Inventor : **Kifune, Koji, c/o Unitika Ltd.**
**Central Research Institute, 23 Unji Kozakura**
**Uji-shi, Kyoto (JP)**
Inventor : **Tanimoto, Nobuyuki, c/o Unitika Ltd.**
**Central Research Institute, 23 Unji Kozakura**
**Uji-shi, Kyoto (JP)**

(74) Representative : **Hansen, Bernd, Dr.rer.nat. et al**
**Hoffmann, Eitle & Partner, Patentanwälte,**
**Postfach 81 04 20**
**D-81904 München (DE)**

## Description

This invention relates to the use of a pharmaceutically effective amount of deacetylated chitin. More particularly, it relates to the use thereof for preparing a medicament for the treatment, in humans, of inflammatory skin diseases, acute eczema, contact dermatitis, atopic dermatitis, chronic eczema, asteatosis, rosacea-like dermatitis, circumoral dermatitis, zona, acne vulgaris, radioactive dermatitis, lepra, lichen planus and erythroderma.

G.G. Allan et al report in their article "Biomedical Applications of Chitin and Chitosan" (1984, p. 119-133; Chitin, Chitosan and Related Enzymes, editor: J.P. Zikakis) that chitosan can be used for the treatment, in animals, of some kinds of dermatitis. The dermatitis to be treated is an exogenous dermatitis caused by mites.

A number of drugs have been developed as anti-inflammatory agents and used in practice hitherto. Typical examples of these drugs include adrenocortical hormone preparations (steroid preparations) and non-steroid preparations for external use. These drugs are formulated into an ointment or a cream.

However, none of the known anti-inflammatory agents can achieve a satisfactory therapeutic effect. Furthermore, it is widely known that adrenocortical hormone preparations (steroid preparations) have various side effects, for example, induction of infection, steroid acne and suppression of adrenocortical functions. Therefore, these preparations should be used carefully.

While nonsteroid preparations are less toxic than the above-mentioned steroid ones, the effects of these nonsteroid preparations are limited. Thus, prolonged application of these drugs is not satisfactory.

It has been proposed to apply chitin, which occurs in nature, to organisms. For example, U.S. Patent 4,532,134 and JP-A-61-52872 (corresponding to U.S. Patent 4,699,135) disclose the application of chitin or chitosan to the treatment of a wound (the term "JP-A" as used herein refers to a "published unexamined Japanese patent application"). Further, JP-A-59-27826 and JP-A-63-255294 propose to use chitin or a hydrolyzate thereof as an antitumor agent or an anti-infective agent, while JP-A-63-275507 proposes to use chitin as a thickener for a hair-care composition.

As described above, chitin is used for the treatment of a wound. Chitin is used for this purpose in order to give protective and therapeutic effects on the wound formed by an external physical or mechanical cause. Chitin has not heretofore been applied to treat inflammatory skin diseases. Similarly, none of known antitumor agents, anti-infective agents nor hair-care compositions containing chitin aims at the treatment of inflammatory skin diseases.

It is an object of the present invention to provide an anti-inflammatory composition which exerts in humans excellent therapeutic effects on inflammatory skin diseases without showing any side effects such as those observed in the case of adrenocortical hormone preparations.

Accordingly, the present invention resides in the use of a pharmaceutically effective amount of deacetylated chitin or a pharmaceutically acceptable salt thereof for preparing a medicament for the treatment, in humans, of inflammatory skin diseases selected from acute excema contact dermatitis, atopic dermatitis, chronic eczema, asteatosis, rosacea-like dermatitis, circumoral dermatitis, zona, acne vulgaris, radioactive dermatitis, lepra, lichen planus and erythroderma.

The anti-inflammatory composition of the present invention exerts an excellent therapeutic effect on inflammatory skin diseases without showing any side effects. Thus, it can be safely used and is highly effective in the treatment of these diseases.

"Chitin" inherently means poly(N-acetyl-D-glucosamine) obtained by treating the external skeleton of a crustacean or an insect with caustic soda to thereby remove the ash and proteins contained therein. However, the term "chitin" as used herein also includes chitin derivatives wherein the -OH or -$CH_2OH$ group of the glucosamine residue is, for example, esterified, etherified, carboxymethylated, hydroxyethylated or 0-ethylated.

It is preferable that the aminoacetyl group in the deacetylated chitin contained in the anti-inflammatory composition of the present invention is deacetylated to a degree of 40% or more, still preferably 60 to 95% and most preferably 65 to 80%. When the degree of deacetylation thereof is lower than 40% by weight, it is sometimes necessary to increase the dose of the anti-inflammatory composition of the present invention.

The deacetylated chitin contained in the anti-inflammatory composition of the present invention may be a pharmaceutically acceptable salt obtained from an amino group and an acid. Examples thereof include acetate, hydrochloride, nitrate or phosphate.

The chitin may be deacetylated by a known method which comprises treating chitin with an alkali. The degree of deacetylation thereof may be readily controlled by appropriately selecting the concentration of the alkali to be used in the deacetylation, treating temperature and treating time.

The degree of deacetylation as used herein is determined by the following method.

Approximately 2 g of a sample is added to 200 ml of a 2 N aqueous solution of hydrochloric acid and stirred at room temperature for 30 minutes. Then, the resulting material is filtered through a glass filter and the filtrate is added to 200 ml of methanol followed by stirring for 30 minutes. Next, the obtained material is further filtered through a glass filter and the filtrate is added to 200 ml of fresh methanol followed by stirring for 30 minutes. This methanol-washing is repeated four times and then the material is air-dried and dried under reduced pressure. Approximately 0.2 g of the dried material is precisely weighed and introduced into a 100 ml Erlenmeyer flask. After adding 40 ml of deionized water, the mixture is stirred for 30 minutes. Then, the obtained solution is subjected to neutralization titration with a 0.1 N aqueous solution of caustic soda by using phenolphthalein as an indicator. The degree of deacetylation (A) may be calculated according to the following equation:

$$A\,(\%) = \frac{2.03 \times f \times b \times 10^{-2}}{a + 0.055 \times f \times b \times 10^{-2}} \times 100$$

wherein a is the weight (g) of the sample; f is the titer of the 0.1 N aqueous solution of caustic soda; and b is the weight (g) of the titrated 0.1 N aqueous solution of caustic soda.

The molecular weight of the deacetylated chitin is not particularly limited. Namely, a deacetylated chitin of from an oligomer to one having a high molecular weight may be effectively used. The preferred molecular weight of the deacetylated chitin is 1,000,000 or less, more preferably 500,000 or less, most preferably 300,000 or less. The molecular weight as used herein is determined by a light-scattering using a solution composed of a chitosan obtained from a chitin, 8.5% formic acid and 0.5 M sodium formate.

The form of the deacetylated chitin thus obtained is not particularly restricted. Thus, it may be in the form of, for example, a powder, granules or fine fibers.

Examples of the dosage form of the anti-inflammatory composition of the present invention include those for internal use such as a tablet, granules, capsule, powder, suspension and solution, as well as those for external use such as ointment or cream. These forms for internal use may be produced by a known method with the use of the deacetylated chitin, as the active ingredient, optionally together with various aids, for example, an excipient, filler, dispersing agent, emulsifier and syrup. In addition, any ointment base specified in The Pharmacopoea of Japan may be used, so long as it has such a spreadability at room temperature as to be easily applied to the skin and would be softened or melt at body temperature. Examples thereof include fats, fatty acids, lanolin, vaseline, glycerol, waxes, resins, plaster preparations, higher alcohols, glycols and surfactants. Either one of these materials or a mixture thereof may be employed. As a cream base, a thick emulsion obtained by mixing fats and water may be employed.

The blending may be conducted in a conventional manner, for example, kneading. Furthermore, other drug(s) such as antibiotic may be added, if required.

The content of the deacetylated chitin in these preparations would vary depending on the condition or dosage form. Generally speaking, it may range from 0.3 to 20% by weight, preferably 1 to 7% by weight and still preferably 2 to 6% by weight. When the content of the deacetylated chitin exceeds the upper limit as defined above, the formulation would become difficult and/or the flowability of the resulting ointment or cream would become undesirably low, which makes the application of the product difficult. When it is smaller than the lower limit as defined above, on the other hand, the obtained product would show only a limited therapeutic effect.

The anti-inflammatory composition of the present invention formulated for internal use may be orally administered. On the other hand, that formulated into an ointment or a cream may be directly applied on the affected part in an appropriate amount and then additionally applied over time. The dose of the anti-inflammatory composition of the present invention for internal use may vary depending on the conditions and dosage form. Generally speaking, it may be administered in a dose of 0.05 to 10 g/day, more preferably 0.1 to 5 g/day, most preferably 0.2 to 3 g/day, of the active ingredient.

The anti-inflammatory composition of the present invention is effective in the treatment of commonly observed inflammatory skin diseases, for example, dermatitis caused by a chemical or a material originating from an animal or a plant and allergic or bacterial infectious dermatitis, and it is used for the treatment of inflammatory skin diseases selected from acute eczema, contact dermatitis, atopic dermatitis, chronic eczema, asteatosis, rosacea-like dermatitis, circumoral dermatitis, zona, acne vulgaris, radioactive dermatitis, lepra, lichen planus and erythroderma.

The acute toxicity of the anti-inflammatory composition of the present invention having a degree of deacetylation of 70% was examined. When dissolved in a physiological saline solution and intraperitoneally administered to a mouse, it shows an $LD_{50}$ value, calculated by the Litchfield-Wilcoxon method, of 7.5 g/kg or above, which indicates that it is highly safe.

To further illustrate the present invention, the following Examples will be given.

EXAMPLE 1 AND COMPARATIVE EXAMPLE 1

A crude chitin powder (manufactured by Shin Nippon Kagaku K.K.) was ground to thereby give particles passing through a 100-mesh sieve. Then, the product was treated with 1 N hydrochloric acid at 4°C for 1 hour. Further, it was treated by heating in a 3% aqueous solution of caustic soda to 90°C for 3 hours. Thus, the calcium and protein contained in the crude chitin powder were removed. The degree of deacetylation of the chitin powder was 5.2%. Next, it was further treated by heating in a 30% aqueous solution of caustic soda to 80°C for 3 hours to thereby deacetylate the same. After repeatedly washing with water and drying, deacetylated chitin was obtained. The degree of deacetylation of the deacetylated chitin powder was 71.2%. Subsequently, the deacetylated chitin thus obtained was mixed with official white petrolatum in such a manner as to give a content of 4% by weight and thoroughly kneaded. Thus, an ointment was obtained (Example 1). Further, this ointment was sterilized in an autoclave (121°C) for 15 minutes. This ointment (Example 1) and another white petrolatum ointment containing 0.1% by weight of dexamethasone propionate which is a steroid preparation (Comparative Example 1) were used in treating atopic dermatitis. The subject used in this test was a woman aged 16 years suffering from atopic dermatitis. She showed a large amount of atopic red exanthemata in the face and both arms. Each affected part was sterilized with, for example, ethyl alcohol and washed. Then, the ointment of Example 1 was applied on the face and the left arm, while that of the Comparative Example 1 was applied on the right arm, each in a sufficient amount. Subsequently, each ointment was wiped away and the affected part was cleaned with ethyl alcohol. Next, the same ointment was applied thereto. This procedure was repeated every day. As a result, the part, to which the ointment of Example 1 had been applied, showed a slow decrease in the red exanthemata from the 5th day. On the 10th day, all of the exanthemata disappeared from the face and the left arm, showing good healing. In contrast thereto, the exanthemata on the right arm, to which the ointment of Comparative Example 1 had been applied, did not disappear, showing no healing.

EXAMPLE 2

The same chitin powder as the one used in Example 1 was deacetylated by treating in a 35% aqueous solution of caustic soda at 80°C for 3 hours. After thoroughly washing with water, the material was dried. The degree of deacetylation of the product thus obtained was 80.3%. The deacetylated chitin was added to an equivalent mixture of beeswax and glycerol in such an amount as to give a content of 8% by weight. Thus, an ointment was prepared. This ointment was then used in the treatment of contact dermatitis. The subject employed in this test was a man aged 21 years who showed exanthemata caused by contact dermatitis over the face. His face was thoroughly sterilized with ethyl alcohol and cleaned. Then, the ointment was applied thereto. Next, the ointment was wiped away and the face was cleaned with ethyl alcohol. Subsequently, the same ointment was applied to each affected part. This procedure was repeated every day. As a result, the exanthemata on the face, to which the ointment had been applied, completely disappeared by the 12th day.

EXAMPLE 3 AND COMPARATIVE EXAMPLE 2

The same chitin powder as the one used in Example 1 was deacetylated by treating in a 25% aqueous solution of caustic soda at 80°C for 3 hours. After thoroughly washing with water, the material was dried. The degree of deacetylation of the product thus obtained was 62.4%. The deacetylated chitin was added to a thick emulsion prepared from a vegetable oil and water in such an amount as to give a concentration of 2% by weight. Thus, a cream (Example 3) was obtained. This cream was used in the treatment of chronic eczema of a man aged 35 years. The ointment of Example 3 was applied to his left thigh which was an affected part. For comparison, an O/W type hydrophilic base cream (Staderm Cream®, produced by Torii Pharmaceutical Co.) containing 5% by weight of ibuprofen piconol which is an anti-inflammatory agent (Comparative Example 2) was applied to his right thigh which was another affected part. Subsequently, the same procedure as those described in the above Examples was repeated every day. As a result, the exanthemata on the left thigh, to which the cream of Example 3 had been applied, slowly disappeared from the 7th day. On the 15th day, the disease was completely healed. In contrast thereto, the right thigh, to which the cream of Comparative Example 2 had been applied, showed no decrease in the exanthemata even on the 15th day.

EXAMPLE 4

A crude chitin powder (manufactured by Shin Nippon Kagaku K.K.) was ground to thereby give particles passing through a 60-mesh sieve. Then, the product was treated with 1 N hydrochloric acid at room temperature for 1.5 hours. Further, it was treated by heating in a 4% aqueous solution of caustic soda to 95°C for 3 hours. Thus, the calcium and protein contained in the crude chitin powder were removed. The degree of deacetylation of the chitin powder was 6.8%. Next, it was further treated by heating in a 42% aqueous solution of caustic soda to 95°C for 1 hour to thereby deacetylate the same. After repeatedly washing with water and drying, deacetylated chitin was obtained. The degree of deacetylation of the deacetylated chitin powder was 45.6%. Subsequently, the deacetylated chitin thus obtained was treated in 2 N acetic acid for 30 minutes and then washed with methanol for 1 hour followed by drying. Then, the powdery deacetylated chitin acetate thus obtained was mixed with a paraffin ointment base, which comprised 95% of liquid paraffin and 5% of a polyethylene resin, in such an amount as to give a content of 2% by weight and thoroughly kneaded. Thus, an ointment was obtained (Example 4). Further, this ointment was sterilized in an autoclave (121°C) for 15 minutes. This ointment (Example 4) was used in treating lepra accompanied by erythroderma. The subject employed in this test was a man aged 52 years showing lepra around the breast along the body axis. Prior to the application of the cream of Example 4, a steroid ointment had been used in the treatment of this subject. However, this treatment had not achieved a satisfactory effect but rather worsened the disease by causing steroid erubescence. The ointment of the present invention was applied twice a day. Then, the erythroderma was improved from the 20th day of the treatment and normal skin was formed. After one month, the normal skin increased, showing a high therapeutic effect. After 40 days, the disease was almost completely healed.

EXAMPLE 5

A crude chitin powder (manufactured by Shin Nippon Kagaku K.K.) was ground to thereby give particles passing through a 150-mesh sieve. Then, the product was treated with 1 N hydrochloric acid at room temperature for 1.5 hours. Further, it was treated by heating in a 4% aqueous solution of caustic soda to 95°C for 3 hours. Thus, the calcium and protein contained in the crude chitin powder were removed. Next, it was further treated by heating in a 42% aqueous solution of caustic soda to 120°C for 1 hour to thereby deacetylate the same. After repeatedly washing with water and drying, deacetylated chitin was obtained. The degree of deacetylation of the deacetylated chitin powder was 83.4%. This powder was used as such in the treatment of zona. Namely, this chitin powder was administered to a patient aged 43 years in a dose of 0.5 g/day every day. Thus, the affected part was improved after 2 weeks. After 1 month, the disease was completely healed, showing an excellent result. During this treatment, no side effect was observed.

**Claims**

1. The use of a pharmaceutically effective amount of deacetylated chitin or a pharmaceutically acceptable salt thereof for preparing a medicament for the treatment, in humans, of inflammatory skin diseases selected from acute exzema, contact dermatitis, atopic dermatitis, chronic eczema, asteatosis, rosacea-like dermatitis, circumoral dermatitis, zona, acne vulgaris, radioactive dermatitis, lepra, lichen planus and erythroderma.

2. The use as claimed in claim 1, wherein said deacetylated chitin or its salt is in a dosage form adapted for administration of an amount of from 0.05 to 10 g/day.

3. The use as claimed in claim 1 or 2, wherein said deacetylated chitin or its salt shows a degree of deacetylation of 40% or more.

4. The use as claimed in any one of claims 1 to 3, wherein said deacetylated chitin or its salt shows a degree of deacetylation of from 60% to 95%.

5. The use as claimed in any one of claims 1 to 4, wherein said deacetylated chitin or its salt shows a degree of deacetylation of 65% to 80%.

6. The use as claimed in any one of claims 1 to 5, wherein said pharmaceutically effective amount is 0.3% to 20% by weight.

7. The use as claimed in any one of claims 1 to 6, wherein said pharmaceutically effect amount is 1% to 7% by weight.

**Patentansprüche**

1. Verwendung einer pharmazeutisch wirksamen Menge eines deacetylierten Chitins oder eines pharmazeutisch annehmbaren Salzes davon zur Herstellung eines Medikaments zur Behandlung beim Menschen von entzündlichen Hautkrankheiten, ausgewählt aus akutem Ekzem, Kontaktdermatitis, atopischer Dermatitis, chronischem Ekzem, Asteatose, rosaceaähnlicher Dermatitis, zirkumoraler Dermatitis, Gürtelrose, Acne vulgaris, radioaktiver Dermatitis, Lepra, Lichen planus und Erythrodermie.

2. Verwendung nach Anspruch 1, worin das deacetylierte Chitin in einer für die Verabreichung einer Menge von 0,05 bis 10 g/Tag eingestellten Dosierungsform vorliegt.

3. Verwendung nach Anspruch 1 oder 2, worin das deacetylierte Chitin oder sein Salz einen Deacetylierungsgrad von 40 % oder mehr zeigt.

4. Verwendung nach einen der Ansprüche 1 bis 3, worin das deacetylierte Chitin oder sein Salz einen Deacetylierungsgrad von 60 bis 95 % zeigt.

5. Verwendung nach einem der Ansprüche 1 bis 4, worin das deacetylierte Chitin oder sein Salz einen Deacetylierungsgrad von 65 bis 80 % zeigt.

6. Verwendung nach einem der Ansprüche 1 bis 5, worin die pharmazeutisch wirksame Menge 0,3 bis 20 Gew.-% ist.

7. Verwendung nach einem der Ansprüche 1 bis 6, worin die pharmazeutisch wirksame Menge 1 bis 7 Gew.-% ist.

**Revendications**

1. Emploi d'une quantité pharmaceutiquement efficace de chitine désacétylée ou d'un sel pharmaceutiquement acceptable de cette dernière pour la préparation d'un médicament destiné au traitement de dermatopathies inflammatoires humaines sélectionnées parmi l'eczéma aigu, la dermatite de contact, la dermatite atopique, l'eczéma chronique, l'astéatose, l'acné rosacée, la dermatite péri-orale, le zona, l'acné vulgaire, la radiodermite, la lèpre, le lichen plan et l'érythrodermie.

2. Emploi selon la revendication 1, dans lequel ladite chitine désacétylée ou son sel se présente sous une forme posologique convenant à l'administration d'une quantité comprise entre 0,05 et 10 g/j.

3. Emploi selon la revendication 1 ou 2, dans lequel ladite chitine désacétylée ou son sel présente un degré de désacétylation supérieur ou égal à 40 %.

4. Emploi selon l'une quelconque des revendications 1 à 3, dans lequel ladite chitine désacétylée ou son sel présente un degré de désacétylation compris entre 60 % et 95 %.

5. Emploi selon l'une quelconque des revendications 1 à 4, dans lequel ladite chitine désacétylée ou son sel présente un degré de désacétylation compris entre 65 % et 80 %.

6. Emploi selon l'une quelconque des revendications 1 à 5, dans lequel ladite quantité pharmaceutiquement efficace est comprise entre 0,3 % et 20 % en poids.

7. Emploi selon l'une quelconque des revendications 1 à 6, dans lequel ladite quantité pharmaceutiquement efficace est comprise entre 1 et 7 % en poids.